(19) Europäisches Patentamt
European Patent Office
Office européen des brevets

(11) Veröffentlichungsnummer: **0 165 540**
**B1**

(12) # EUROPÄISCHE PATENTSCHRIFT

(45) Veröffentlichungstag der Patentschrift: **16.12.87**

(51) Int. Cl.⁴: **C 07 C 51/363**, C 07 C 53/19

(21) Anmeldenummer: **85107159.7**

(22) Anmeldetag: **11.06.85**

(54) **Verfahren zur Herstellung von 2-Chlorpropionsäure.**

(30) Priorität: **19.06.84 DE 3422672**

(43) Veröffentlichungstag der Anmeldung: **27.12.85 Patentblatt 85/52**

(45) Bekanntmachung des Hinweises auf die Patenterteilung: **16.12.87 Patentblatt 87/51**

(84) Benannte Vertragsstaaten: **BE CH DE FR GB IT LI NL**

(56) Entgegenhaltungen:
**DE-A-936 443**
**GB-A-621 531**
**GB-A-793 912**

(73) Patentinhaber: **HOECHST AKTIENGESELLSCHAFT, Postfach 80 03 20, D-6230 Frankfurt am Main 80 (DE)**

(72) Erfinder: **Landauer, Franz, Dr., Liederbacher Strasse 7, D-6230 Frankfurt am Main 80 (DE)**

**EP 0 165 540 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

2-Chlorpropionsäure $CH_3CHClCOOH$ ist ein wertvolles Zwischenprodukt insbesondere zur Herstellung von Pflanzenschutzmitteln, Pharmazeutika und Farbstoffen.

Es ist bekannt, 2-Chlorpropionsäure durch Chlorierung von Propionsäure bei 110 bis 120°C in Gegenwart von Katalysatoren herzustellen; als Katalysatoren dienen vorwiegend P- und S-haltige Verbindungen ($PCl_3$ $S_2Cl_2$, $ClSO_3H$, etc.), u.a. aber auch Propionylchlorid (vgl. Ullmanns Enzyklopädie der technischen Chemie, 4. Aufl., Bd. 19 (1980) S. 457/458).

Dem Propionylchlorid-Katalysator kann gegebenenfalls auch noch Propionsäureanhydrid zugesetzt werden (GB-PS-892 584). Für die Monochlorierung der Propionsäure sind in der GB-PS Temperaturen zwischen 100 und 110°C angegeben. Bei höherer Temperatur (150 bis 160°C) soll dann die Dichlorierung zur 2,2-Dichlorpropionsäure stattfinden.

Die vorerwähnte GB-PS enthält auch eine Vorstellung über den Mechanismus der Chlorierung der Propionsäure in 2-Stellung. Danach soll die Reaktion über tautomeres Propionylchlorid nach folgendem Reaktionsschema (für die Monochlorierung) verlaufen:

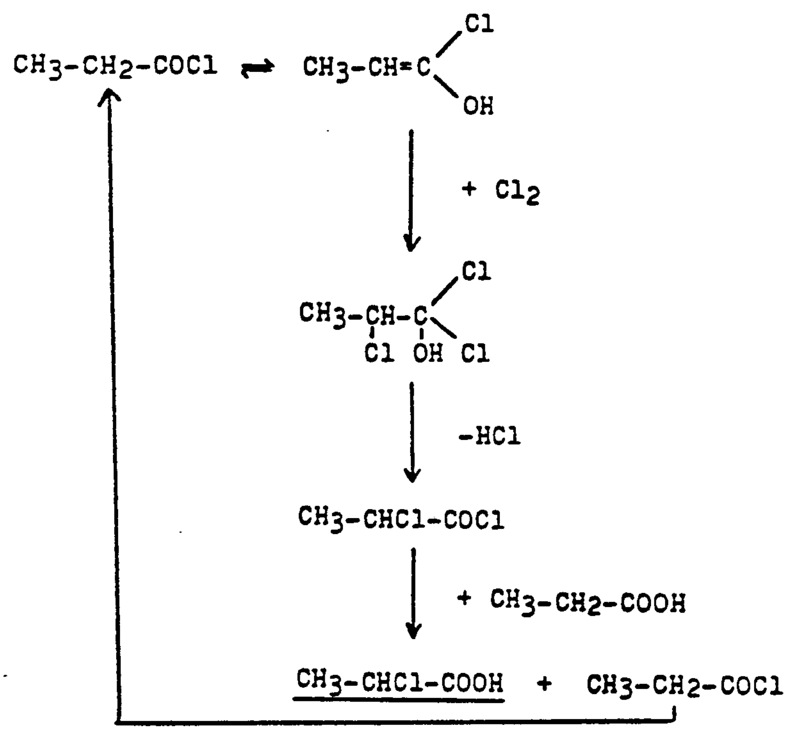


Wenn die Chlorierung der Propionsäure in 2-Stellung beabsichtigt ist, sind Radikalbildner und Licht möglichst auszuschließen, da die Chlorierung unter radikalbildenden Bedingungen vorwiegend in 3-Stellung (unter Bildung der 3-Chlorpropionsäure $CH_2Cl$-$CH_2$-COOH) erfolgt.

Nach den Untersuchungen von Y. Ogata und K. Matsuyama über den Einfluß von Katalysatoren auf die Chlorierung von Propionsäure (vgl. Tetrahedron, Vol. 26 (1970), S. 5929-5937) kann als Zwischenprodukt für die Chlorierung der Propionsäure in 2-Stellung auch die tautomere Propionsäure selbst in Frage kommen:

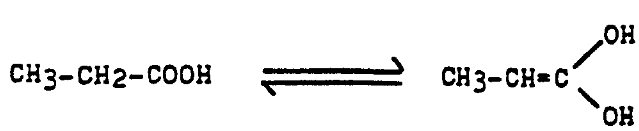

Die Autoren berichten auch über Versuche der Monochlorierung von Propionsäure bei 110°C u.a. mit Propionsäureanhydrid als alleinigem Katalysator während einer 3-stündigen Reaktionsdauer (siehe Tabelle 2 auf Seite 5931). In Gegenwart von 4 Mol-% Propionsäureanhydrid (bezogen auf die Ausgangs-Propionsäure) soll das Reaktionsprodukt ein Verhältnis von 2-Chlorpropionsäure ("α")/2-Chlorpropionsäure ("α") + 3-Chlorpropionsäure ("β") von 0,38 enthalten haben.

In Gegenwart von 17 Mol-% Propionsäureanhydrid soll das Verhältnis $\alpha/\alpha+\beta$ 0,71 betragen haben.

In eigenen Versuchen der Monochlorierung von Propionsäure bei etwa 110°C in Gegenwart von 8 Mol-% Propionsäureanhydrid bei einer Reaktionsdauer von 15 Stunden wurde ohne Lichtausschluß ein Verhältnis von

$\alpha/\alpha+\beta = 0,76$ erhalten, unter Lichtausschluß ein Verhältnis von $\alpha/\alpha+\beta = 0,92$.

Die Möglichkeit der Chlorierung von Propionsäure in Gegenwart von Propionsäureanhydrid ist auch in der älteren GB-PS-621 531 erwähnt; nähere Details hierüber enthält die Patentschrift - die sich in erster Linie auf die Chlorierung von Essigsäure bezieht - jedoch nicht. Der für die Chlorierung von Essigsäure (in Gegenwart von Essigsäureanhydrid oder -chlorid) angegebene Temperaturbereich liegt bei 100 bis 110°C.

Die bekannten Verfahren zur Herstellung der 2-Chlorpropionsäure sind in verschiedener Hinsicht nicht ganz befriedigend. Im Falle der Verwendung etwa der Phosphor- und Schwefelverbindungen als Katalysatoren entstehen z.T. schwer-lösliche P- bzw. S-haltige harzartige Produkte, welche eine relativ häufige und aufwendige Reinigung der Chlorierungsapparatur erfordern; bei kontinuierlichem Prozeß kann dies zu häufigen Betriebsunterbrechungen führen. Außerdem müssen die P- und S-haltigen Rückstände auch in umweltschonender Weise aufgearbeitet werden. Schließlich gelangen in die nach diesen Verfahren hergestellte 2-Chlorpropionsäure häufig noch geringe Mengen an P- oder S-Verbindungen als Verunreinigungen, die die Qualität der 2-Chlorpropionsäure beeinträchtigen.

Für den Einsatz des Propionylchlorids (Kp 80°C) als Katalysator ist nachteilig, daß diese Verbindung bei der Chlorierungstemperatur vom entstehenden Chlorwasserstoff leicht zumindest teilweise ausgestrippt wird und damit bei der Reaktion fehlt, bzw. den Chlorwasserstoff verunreinigt und dessen Wiederverwendung erschwert.

Im Falle der Verwendung des Propionsäureanhydrids als Katalysator ist insbesondere ausweislich der Versuche von Y. Ogata und K. Matsuyama bei 110°C das $\alpha/\alpha+\beta$-Verhältnis jedenfalls für höhere industrielle Anforderungen nicht ausreichend höhere Temperaturen begünstigen nach der gleichen Literaturtstelle den Radikalmechanismus und damit die Bildung der 3-Chlorpropionsäure, was eine weitere Erniedrigung des $\alpha/\alpha+\beta$-Verhältnisses erwarten läßt.

Es bestand daher die Aufgabe, ein verbessertes Verfahren zur Herstellung von 2-Chlorpropionsäure zu finden.

Diese Aufgabe konnte erfindungsgemäß dadurch gelöst werden, daß man Propionsäure in Gegenwart von Propionsäureanhydrid als alleinigem Katalysator oder Zusatzstoff unter Vermeidung radikalbildender Bedingungen bei Temperaturen chloriert, welche über den bisher für die Chlorierung von Propionsäure zu 2-Chlorpropionsäure in Gegenwart von Propionsäureanhydrid angewandten Temperaturen (etwa 110°C) liegen.

Erfindungsgegenstand ist daher ein Verfahren zur Herstellung von 2-Chlorpropionsäure durch Chlorierung von Propionsäure in Gegenwart von Propionsäureanhydrid bei erhöhter Temperatur unter Ausschluß von Radikalbildnern und von Licht,

das dadurch gekennzeichnet ist, daß man die Chlorierung ohne einen weiteren Zusatzstoff bei Temperaturen zwischen 115 und 140°C durchführt. Bevorzugt sind Temperaturen zwischen etwa 120 und etwa 135°C, inbesondere zwischen etwa 125 und 135°C.

Bei diesen höheren Temperaturen werden bei hohen bis quantitativen Umsätzen Ausbeuten an 2-Chlorpropionsäure über 90 % d.Th. bei einem $\alpha/\alpha+\beta$-Verhältnis durchweg zwischen etwa 0,99 und 1,0 erhalten.

Dieses Ergebnis war außerordentlich überraschend, da man bei höheren Temperaturen infolge der Begünstigung der Bildung von Radikalen mit zunehmender Temperatur eher eine Zunahme des Anteils an (der hier unerwünschten) 3-Chlorpropionsäure - die bei der Chlorierung von Propionsäure unter radikalbildenden Bedingungen bevorzugt entsteht - und damit ein niedrigeres $\alpha/\alpha+\beta$-Verhältnis erwarten mußte.

Die Menge des nach dem erfindungsgemäßen Verfahren zugesetzten Propionsäureanhydrids liegt im allgemeinen zwischen etwa 3 und etwa 30 Mol-%, vorzugsweise zwischen etwa 5 und etwa 20 Mol-%, bezogen auf die Ausgangs-Propionsäure. Höhere Zusatzmengen sind möglich, bringen aber keinen Vorteil. Andere Katalysatoren und/oder Zusatzstoffe sollen bei dem Verfahren nicht mit verwendet werden. Außerdem ist auch darauf zu achten, daß Radikalbildner und Licht ausgeschlossen bleiben.

Ansonsten wird das Verfahren in üblicher Weise in für Chlorierungen geeigneten Apparaturen unter Lichtausschluß durchgeführt. Sowohl eine diskontinuierliche als auch eine kontinuierliche Verfahrenweise ist möglich.

Weiterhin läßt sich das Verfahren sowohl unter Normal- als auch unter Überdruck durchführen, wobei die Verfahrensweise unter Normaldruck bevorzugt ist.

Das für die Chlorierung verwendete Chlor kann mit gegenüber den Ausgangs- und Endstoffen der Reaktion inerten Gasen wie z. B. Stickstoff oder Chlorwasserstoff verdünnt werden.

Auch der Einsatz von inerten Lösemitteln wie z. B. von Chlorkohlenwasserstoffen (Chlorbenzol etc.) ist möglich.

Wegen der nach dem erfindungsgemäßen Verfahren erzielten hohen Ausbeute und Reinheit der 2-Chlorpropionsäure sowie wegen der Vermeidung schwer abtrennbarer Katalysatoren und auch weil der hier verwendete Katalysator wegen seines relativ hohen Siedepunktes nicht etwa ebenso leicht wie z. B. Propionylchlorid vom entstehenden Chlorwasserstoff mit ausgestrippt werden kann, stellt die Erfindung einen erheblichen Fortschritt dar.

Die nachfolgenden Beispiele dienen der näheren Erläuterung der Erfindung. Nach den Erfindungsbeispielen (A) folgen noch zwei Vergleichsbeisplele (B), welche in Analogie zu den Versuchen von Y. Ogata und K. Matsuyama (a.a.O.) bei etwa 110°C ohne und unter Lichtausschluß durchgeführt wurden.

A)

**Erfindungsbeispiele:**

Beispiel 1:

In einem 1-Liter-Reaktor, versehen mit Rückflußkühler, Rührer, Thermometer und Gaseinleitungsrohr wurden 370 g Propionsäure (= 5 Mol) und 104 g (= 0,8 Mol = 16 Mol-%, bezogen auf die Propionsäure) Propionsäureanhydrid auf etwa 130°C erhitzt. Unter Ausschluß von Licht wurden dann im Verlauf von 20 Stunden 470 g Chlor (= 6,62 Mol) eingeleitet. Nach Ausblasen des Chlorwasserstoffs mit Stickstoff wurden 691 g Ausbeute erhalten.

Die gaschromatographische Analyse ergab (nach Verseifen mit Wasser, um restliches chloriertes Propionsäureanhydrid In die Chlorpropionsäure zu überführen):

| | |
|---|---|
| Propionsäure: | 1,3 % |
| 2-Chlorpropionsäure("α"): | 97,5 % |
| 3-Chlorpropionsäure ("β"): | 0,1 % |
| 2,2-Dichlorpropionsäure: | 0,7 % |
| α/α+β: = | 0,998 |

Beispiel 2:

Nach der Verfahrensweise des Beispiels 1 wurden 370 g Propionsäure (5 Mol) und 52 g Propionsäureanhydrid (= 0,4 Mol =8 Mol-%, bezogen auf die Propionsäure) bei etwa 120°C chloriert, wobei im Verlauf von 15 Stunden 412 g Chlor (=5,8 Mol) eingeleitet wurden. Eine Probe ergab (wiederum nach Verseifen mit Wasser):

| | |
|---|---|
| Propionsäure: | 5,7 % |
| 2-Chlorpropionsäure ("α"): | 92,0 % |
| 3-Chlorpropionsäure ("β"): | 0,6 % |
| 2,2-Dichlorpropionsäure: | 1,6 % |
| α/α+β = | 0,993 |

B)

**Vergleichsbeispiele**

Vergleichsbeispiel 1

In einem 1-Liter-Vierhalskolben mit Rückflußkühler, Rührer, Thermometer und Gaseinleitungsrohr wurden ohne Lichtausschluß 370 g Propionsäure (5 Mol) und 52 g Propionsäureanhydrid (0,4 Mol = 8 Mol-%, bezogen auf die Propionsäure) bei etwa 110°C chloriert, wobei im Verlauf von 15 Stunden 391 g Chlor (= 5,5 Mol) eingeleitet wurden.

Eine Probe ergab (wiederum nach Verseifen mit Wasser) bei der GC-Analyse:

| | |
|---|---|
| Propionsäure: | 11,5 % |
| 2-Chlorpropionsäure ("α"): | 63,4 % |
| 3-Chlorpropionsäure ("β"): | 20,0 % |
| 2,2-Dichlorpropionsäure: | 3,6 % |
| α/α+β = | 0,76 |

Vergleichsbeispiel 2

Vergleichsbeispiel 1 wurde unter Lichtausschluß (unter ansonsten identischen Bedingungen) wiederholt. Dabei wurden folgende GC-Werte erhalten:

| | |
|---|---|
| Propionsäure: | 5,2 % |
| 2-Chlorpropionsäure: | 82,8 % |
| 3-Chlorpropionsäure: | 7,2 % |
| 2,2-Dichlorpropionsäure: | 4,8 % |
| α/α+β = | 0,92 |

**Patentansprüche**

1. Verfahren zur Herstellung von 2-Chlorpropionsäure durch Chlorierung von Propionsäure in Gegenwart von Propionsäureanhydrid bei erhöhter Temperatur unter Ausschluß von Radikalbildnern und von Licht, dadurch gekennzeichnet, daß man die Chlorierung ohne einen welteren Zusatzstoff bei Temperaturen zwischen 115 und

140° C, vorzugsweise zwischen etwa 120 und 135° C, insbesondere zwischen etwa 125 und etwa 135° C durchführt.

2. Verfahren nach Anspruch 1, dadurch gekennzeichnet, daß die Menge des zugesetzten propionsäureanhydrids zwischen etwa 3 und etwa 30 Mol-%, vorzugsweise zwischen 5 und etwa 20 Mol-%, bezogen auf die Ausgangs-Propionsäure, beträgt.

## Claims

1. A process for the preparation of 2-chloropropionic acid by chlorinating propionic acid in the presence of propionic anhydride at an elevated temperature with the exclusion of free radical-formers and of light, characterized in that the chlorination is carried out without a further additive at temperatures between 115 and 140° C, preferably between about 120 and 135° C, especially between about 125 and about 135° C.

2. The process as claimed in claim 1, wherein the amount of the propionic anhydride addes is between about 3 and about 30 mol %, preferably between 5 and about 20 mol %, relative to the starting propionic acid.

## Revendications

1. Procédé pour préparer l'acide chloro-2 propionique par chloration de l'acide propionique en présence d'anhydride propionique, à température élevée, en l'absence de générateurs de radicaux et à l'abri de la lumière, procédé caractérisé en ce qu'on effectue la chloration sans autre additif, à des températures comprises entre 115 et 140° C, de préference entre environ 120 et 135° C, plus spécialement entre environ 125 et environ 135° C.

2. Procédé selon la revendication 1 caractérisé en ce que la quantité de l'anhydride propianique ajouté est comprise entre environ 3 et environ 30 % en moles, de préférence entre 5 et entre environ 20 % en moles par rapport à l'acide propionique de depart.